# EUROPEAN PATENT SPECIFICATION

(11) **EP 4 186 438 B1**
(45) Date of publication and mention of the grant of the patent: **02.07.2025**
(21) Application number: 21846513.6
(22) Date of filing: 14.07.2021
(51) Int. Cl.: A61B 8/12, A61B 8/00, B06B 1/06, G02B 23/24, G10K 11/04, G10K 11/30

(54) **ULTRASONIC VIBRATOR UNIT AND ULTRASONIC ENDOSCOPE**
ULTRASCHALLVIBRATOR UND ULTRASCHALLENDOSKOP
UNITÉ DE VIBREUR À ULTRASONS ET ENDOSCOPE À ULTRASONS

(30) Priority: 22.07.2020 JP 2020125267
(43) Date of publication of application: 31.05.2023
(73) Proprietor: FUJIFILM Corporation, Tokyo 106-8620 (JP)
(72) Inventor: MORIMOTO, Yasuhiko, Ashigarakami-gun, Kanagawa 258-8538 (JP); YAMAMOTO, Katsuya, Ashigarakami-gun, Kanagawa 258-8538 (JP); FURUKAWA, Kazushi, Ashigarakami-gun, Kanagawa 258-8538 (JP)
(74) Representative: Dehns Germany Partnerschaft mbB
(86) International application number: PCT/JP2021/026403
(87) International publication number: WO 2022/019186

(56) References cited:
- WO-A1-2018/003322
- JP-A- 2016 120 183
- US-A1- 2019 038 257
- ANONYMOUS: "Anisotropic conductive film - Wikipedia", 25 June 2020 (2020-06-25), XP093096118, Retrieved from the Internet <URL:https://en.wikipedia.org/w/index.php?title=Anisotropic_conductive_film&oldid=964456566> [retrieved on 20231030]

## Description

### BACKGROUND OF THE INVENTION

### 1. Field of the Invention

The present invention relates to an ultrasonic oscillator unit and an ultrasonic endoscope.

### 2. Description of the Related Art

In recent years, an ultrasonic endoscope that observes a state inside a body of a subject by irradiating the inside of the body with ultrasonic waves and receives reflected waves to capture video has been used in medical practice.

In such an ultrasonic endoscope, for example, as disclosed in WO2018/003322A, an ultrasonic oscillator unit is provided in a distal end part of an insertion part that is inserted into a body. In general, the ultrasonic oscillator unit has an ultrasonic oscillator array including a plurality of ultrasonic oscillators (transducers), and the ultrasonic oscillator array is held while being attached to an exterior member provided in the distal end part.

WO2018/003322A discloses a connection structure that electrically connects electrode pads of the ultrasonic oscillators and electrode pads of a flexible printed wiring board (hereinafter, simply referred to as a flexible printed circuit (FPC)) by solder wires or conductive paste (for example, silver paste). Other ends of a plurality of coaxial cables having one ends connected to an ultrasound processor device are electrically connected to the above-described FPC. US 2019/038257 A1 relates to an ultrasonic oscillator unit.

### SUMMARY OF THE INVENTION

Note that, in the connection structure using soldering out of the above-described connection structures, in a case where heat of soldering in providing the solder wires is transmitted to the ultrasonic oscillators, microcracks may occur in the ultrasonic oscillators due to heat. The microcracks cause degradation of quality of an ultrasound image, and it is not preferred. On the other hand, the connection structure using conductive paste can solve a heat problem by soldering, but has the following problem.

That is, the ultrasonic endoscope is put into, for example, a sterilizing tank of a gas sterilization device and is cleaned after being used in an operation. In this case, the ultrasonic endoscope is cleaned through exposure to sterilizing gas, such as ethylene oxide gas or hydrogen peroxide plasma gas, under a reduced pressure atmosphere; however, sterilizing gas has a component that causes change in quality or deterioration of a member with which sterilizing gas is brought into contact. For this reason, in a case where the ultrasonic oscillator unit is cleaned by sterilizing gas many times, an electrical bonded portion formed of, for example, conductive paste may be deteriorated and disconnection may occur.

The present invention has been accomplished in view of such a situation, and an object of the present invention is to provide an ultrasonic oscillator unit and an ultrasonic endoscope capable of suppressing deterioration of an electrical bonded portion due to sterilizing gas.

To achieve the object of the present invention, there is provided an ultrasonic oscillator unit of the present invention according to claim 1 that is disposed in a distal end part of an endoscope insertion part and has a plurality of ultrasonic oscillators, in which the ultrasonic oscillators each have a piezoelectric body, a cable that is electrically bonded to the piezoelectric body is inserted into an internal space of the distal end part, at least one of a plurality of electrical bonded portions from the cable to the piezoelectric body is bonded by a resin material having conductivity (conductive resin material), the electrical bonded portion using the resin material is covered with a first resin layer, and first resin is gas barrier epoxy resin.

In an aspect of the present invention, it is preferable that the epoxy resin has a polyoxyalkylene structure.

In an embodiment of the present invention, it is preferable that the epoxy resin contains an alcohol compound.

In an embodiment of the present invention, it is preferable that a hydroxyl group equivalent of the alcohol compound is equal to or greater than 25 and equal to or less than 150, and a molecular weight of the alcohol compound is equal to or greater than 50 and equal to or less than 500.

In an embodiment of the present invention, it is preferable that the epoxy resin has a polyamide structure.

In an embodiment of the present invention, it is preferable that the internal space of the distal end part into which the cable is inserted is filled with a second resin layer, and second resin is epoxy resin.

In an embodiment of the present invention, it is preferable that the epoxy resin has a polyamide structure.

In an embodiment of the present invention, it is preferable that the first resin layer has higher viscosity before curing than the second resin layer.

To achieve the object of the present invention, there is provided an ultrasonic endoscope of the present invention according to claim 9 having an insertion part that is inserted into a body, an
ultrasonic observation part that is provided at a distal end of the insertion part, and the ultrasonic oscillator unit of the present invention provided in the ultrasonic observation part.

According to the present invention, it is possible to suppress deterioration of an electrical bonded portion due to sterilizing gas.

### BRIEF DESCRIPTION OF THE DRAWINGS

Fig. 1 is a schematic configuration diagram showing an example of the configuration of an ultrasonography system.
Fig. 2 is a partial enlarged plan view showing a distal end part 40 of an ultrasonic endoscope of Fig. 1 and the vicinity of the distal end part.
Fig. 3 is a sectional view taken along the line III-III of Fig. 2.
Fig. 4 is a sectional view taken along the line IV-IV of Fig. 3.
Fig. 5 is a sectional view of a coaxial cable.

### DESCRIPTION OF THE PREFERRED EMBODIMENTS

Hereinafter, a preferred embodiment of an ultrasonic oscillator unit and an ultrasonic endoscope according to the present invention will be described referring to the accompanying drawings.

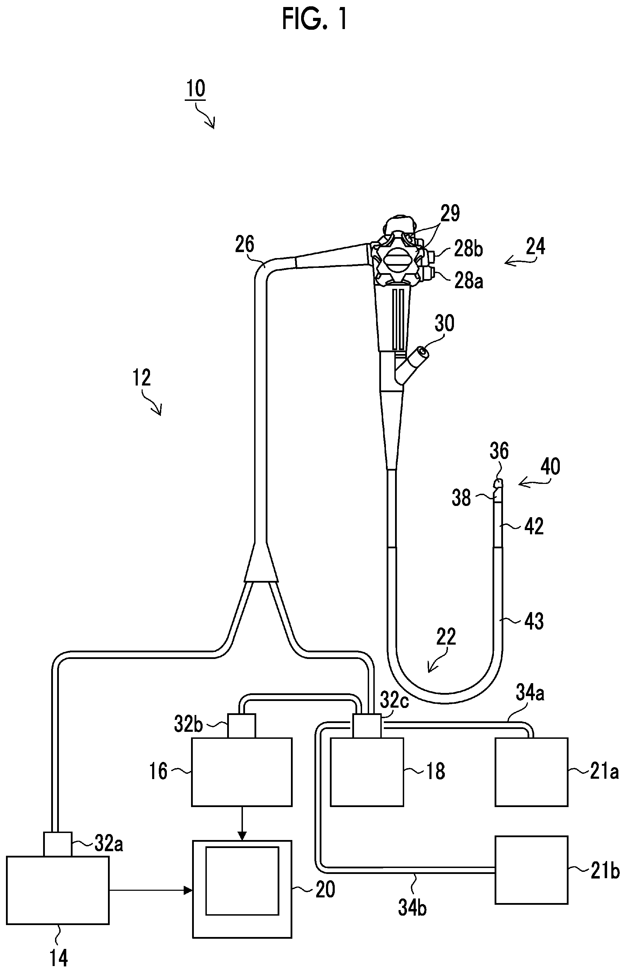

Fig. 1 is a schematic configuration diagram showing an example of an ultrasonography system 10 that uses an ultrasonic endoscope 12 of the embodiment.

As shown in Fig. 1, the ultrasonography system 10 comprises the ultrasonic endoscope 12, an ultrasound processor device 14 that generates an ultrasound image, an endoscope processor device 16 that generates an endoscope image, a light source device 18 that supplies illumination light, with which the inside of a body cavity is illuminated, to the ultrasonic endoscope 12, and a monitor 20 that displays the ultrasound image and the endoscope image. The ultrasonography system 10 comprises a water supply tank 21a that stores cleaning water or the like, and a suction pump 21b that sucks aspirates inside the body cavity.

The ultrasonic endoscope 12 has an insertion part 22 that is inserted into a body cavity of a subject, an operating part 24 that is consecutively provided in a proximal end part of the insertion part 22 and is used by an operator to perform an operation, a universal cord 26 that has one end connected to the operating part 24, and a distal end part 40 of the insertion part 22 comprises an ultrasonic observation part 36 and an endoscope observation part 38 described below.

In the operating part 24, an air and water supply button 28a that opens and closes an air and water supply pipe line (not shown) from the water supply tank 21a, and a suction button 28b that opens and closes a suction pipe line (not shown) from the suction pump 21b are provided sequentially. In the operating part 24, a pair of angle knobs 29 and 29 and a treatment tool insertion port 30 are provided.

In the other end portion of the universal cord 26, an ultrasound connector 32a that is connected to the ultrasound processor device 14, an endoscope connector 32b that is connected to the endoscope processor device 16, and a light source connector 32c that is connected to the light source device 18 are provided. The ultrasonic endoscope 12 is attachably and detachably connected to the ultrasound processor device 14, the endoscope processor device 16, and the light source device 18 respectively through the connectors 32a, 32b, and 32c. The connector 32c comprises an air and water supply tube 34a that is connected to the water supply tank 21a, and a suction tube 34b that is connected to the suction pump 21b.

The insertion part 22 has, in order from a distal end side, the distal end part 40 that has the ultrasonic observation part 36 and the endoscope observation part 38, a bendable part 42 that is consecutively provided on a proximal end side of the distal end part 40, and a soft part 43 that couples a proximal end side of the bendable part 42 and the distal end side of the operating part 24.

The bendable part 42 is remotely bent and operated by rotationally moving and operating a pair of angle knobs 29 and 29 provided in the operating part 24. With this, the distal end part 40 can be directed in a desired direction.

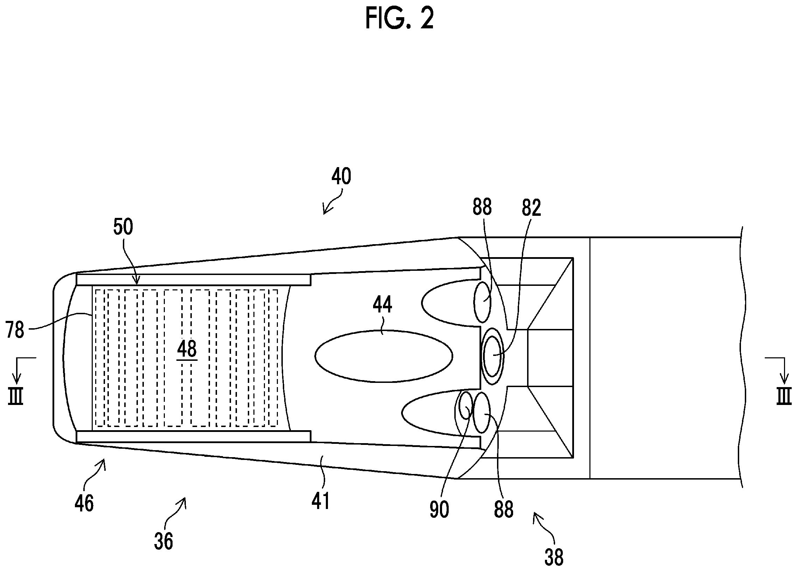

The ultrasound processor device 14 generates and supplies an ultrasound signal for making an ultrasonic oscillator array 50 of an ultrasonic oscillator unit 46 (see Fig. 2) of the ultrasonic observation part 36 described below generate an ultrasonic wave. The ultrasound processor device 14 receives and acquires an echo signal reflected from an observation target part irradiated with the ultrasonic wave, by the ultrasonic oscillator array 50 and executes various kinds of signal processing on the acquired echo signal to generate an ultrasound image that is displayed on the monitor 20.

The endoscope processor device 16 receives and acquires a captured image signal acquired from the observation target part illuminated with illumination light from the light source device 18 in the endoscope observation part 38 and execute various kinds of signal processing and image processing on the acquired image signal to generate an endoscope image that is displayed on the monitor 20.

To image an observation target part inside a body cavity using the endoscope observation part 38 to acquire an image signal, the light source device 18 generates illumination light, such as white light including light of three primary colors of red light, green light, and blue light or light of a specific wavelength. Light propagates through a light guide (not shown) and the like in the ultrasonic endoscope 12, and is emitted from the endoscope observation part 38, and the observation target part inside the body cavity is illuminated with light.

The monitor 20 receives video signals generated by the ultrasound processor device 14 and the endoscope processor device 16 and displays an ultrasound image and an endoscope image. In regard to the display of the ultrasound image and the endoscope image, only one image may be appropriately switched and displayed on the monitor 20 or both images may be displayed simultaneously.

Next, the configuration of the distal end part 40 will be described referring to Figs. 2 to 4.

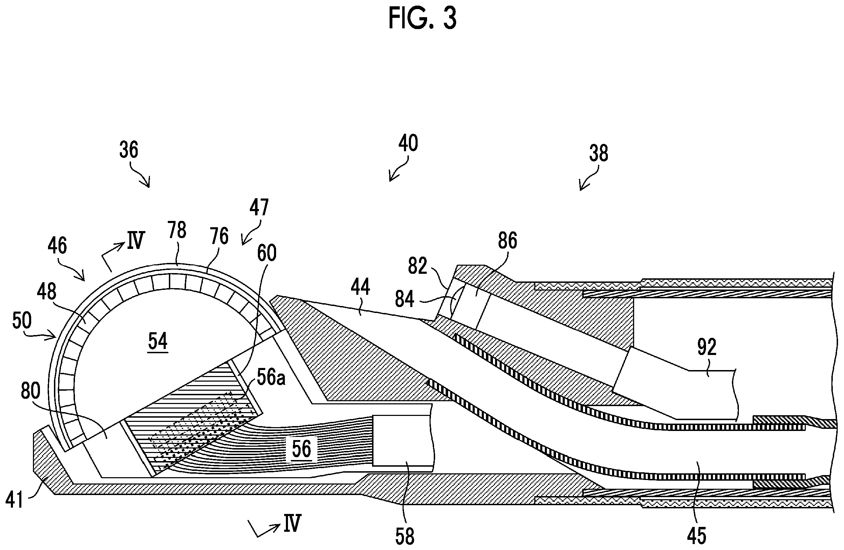

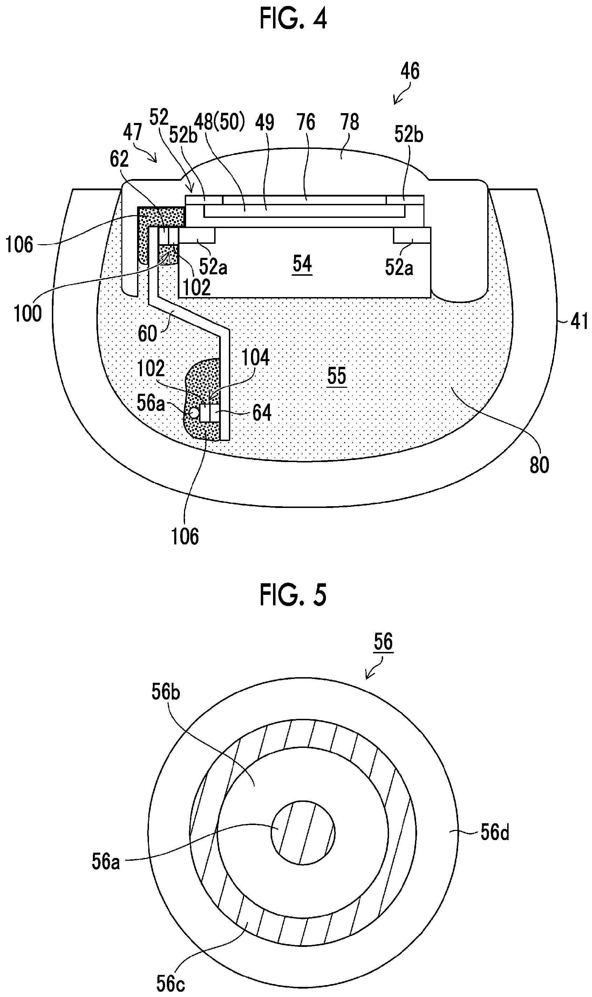

Fig. 2 is a partial enlarged plan view showing the distal end part 40 shown in Fig. 1 and the vicinity thereof the distal end part 40. Fig. 3 is a sectional view taken along the line III-III shown in Fig. 2, and is a longitudinal sectional view of the distal end part 40 taken along a center line thereof in a longitudinal direction. Fig. 4 is a sectional view taken along the line IV-IV shown in Fig. 3, and is a cross-sectional view of the ultrasonic oscillator array 50 of the ultrasonic observation part 36 of the distal end part 40 taken along a center line of an arc structure.

As shown in Figs. 2 and 3, in the distal end part 40, the ultrasonic observation part 36 that acquires the ultrasound image is mounted on the distal end side, the endoscope observation part 38 that acquires the endoscope image is mounted on the proximal end side, and a treatment tool outlet port 44 is provided between the ultrasonic observation part 36 and the endoscope observation part 38.

The endoscope observation part 38 is configured with an observation window 82, an objective lens 84, a solid-state imaging element 86, illumination windows 88, a cleaning nozzle 90, a wiring cable 92 including a plurality of coaxial cables (not shown), and the like.

The treatment tool outlet port 44 is connected to a treatment tool channel 45 that is inserted into the insertion part 22, and a treatment tool (not shown) that is inserted from the treatment tool insertion port 30 of Fig. 1 is led out from the treatment tool outlet port 44 into the body cavity through the treatment tool channel 45.

As shown in Figs. 2 to 4, the ultrasonic observation part 36 comprises the ultrasonic oscillator unit 46, an exterior member 41 that holds the ultrasonic oscillator unit 46, and a plurality of coaxial cables 56 that are wired in the ultrasonic oscillator unit 46. The exterior member 41 is made of a rigid member, such as rigid resin, and configures a part of the distal end part 40.

The ultrasonic oscillator unit 46 has the ultrasonic oscillator array 50 that includes a plurality of ultrasonic oscillators 48, an electrode 52 that is provided on an end portion side of the ultrasonic oscillator array 50 in a width direction (a direction perpendicular to the longitudinal axis direction of the insertion part 22), a backing material layer 54 that supports each ultrasonic oscillator 48 from a lower surface side, an FPC 60 that is disposed along a side surface of the backing material layer 54 in the width direction and is connected to the electrode 52, and a filler layer 80 as a second resin layer with which an internal space 55 between the exterior member 41 and the backing material layer 54 is filled.

The ultrasonic oscillator unit 46 has an acoustic matching layer 76 laminated on the ultrasonic oscillator array 50, and an acoustic lens 78 laminated on the acoustic matching layer 76. That is, the ultrasonic oscillator unit 46 is configured as a laminate 47 having the acoustic lens 78, the acoustic matching layer 76, the ultrasonic oscillator array 50, and the backing material layer 54.

The ultrasonic oscillator array 50 is configured with a plurality of rectangular parallelepiped ultrasonic oscillators 48 arranged in a convex arc shape outward. The ultrasonic oscillator array 50 is an array of 48 to 192 channels consisting of 48 to 192 ultrasonic oscillators 48, for example. Each of the ultrasonic oscillators 48 has a piezoelectric body 49.

The ultrasonic oscillator array 50 of the present example is configured by arranging a plurality of ultrasonic oscillators 48 at predetermined pitches in a one-dimensional array shape as an example. The ultrasonic oscillators 48 that configure the ultrasonic oscillator array 50 are arranged at regular intervals in a convex bent shape along an axial direction of the distal end part 40 (the longitudinal axis direction of the insertion part 22) and are sequentially driven based on drive signals input from the ultrasound processor device 14 (see Fig. 1). With this, convex electronic scanning is performed with a range where the ultrasonic oscillators 48 shown in Fig. 2 are arranged, as a scanning range.

The electrode 52 of the ultrasonic oscillator array 50 has an individual electrode 52a individually and independently provided for each ultrasonic oscillator 48, and an oscillator ground 52b that is a common electrode common to all the ultrasonic oscillators 48. In Fig. 4, a plurality of individual electrodes 52a are disposed on lower surfaces of end portions of a plurality of ultrasonic oscillators 48, and the oscillator ground 52b is provided on upper surfaces of the end portions of the ultrasonic oscillators 48.

The acoustic matching layer 76 is a layer that is provided for taking acoustic impedance matching between the subject and the ultrasonic oscillators 48.

The acoustic lens 78 is a lens that is provided for converging the ultrasonic waves emitted from the ultrasonic oscillator array 50 toward the observation target part. In the acoustic lens 78, powder, such as titanium oxide, alumina, or silica, is mixed as necessary to take acoustic impedance matching between the subject and the ultrasonic oscillators 48 in the acoustic matching layer 76, and to increase the transmittance of the ultrasonic waves. The acoustic lens 78 is formed of, for example, silicon-based resin (millable type silicon rubber, liquid silicon rubber, or the lie), butadiene-based resin, or polyurethane-based resin.

As shown in Figs. 3 and 4, the backing material layer 54 is a layer of a member made of a backing material disposed on an inside with respect to the arrangement surface of a plurality of ultrasonic oscillators 48, that is, a rear surface (lower surface) of the ultrasonic oscillator array 50. The backing material layer 54 has a role of mechanically and flexibly supporting the ultrasonic oscillator array 50 and attenuating ultrasonic waves propagated to the backing material layer 54 side among ultrasound signals oscillated from a plurality of ultrasonic oscillators 48 or reflected and propagated from the observation target. For this reason, the backing material is made of a material having rigidity, such as hard rubber, and an ultrasonic wave attenuation material (ferrite, ceramics, or the like) is added as needed.

The filler layer 80 is a layer with which the internal space 55 between the exterior member 41 and the backing material layer 54 is filled, and has a role of fixing the FPC 60, the coaxial cables 56, and various wiring portions. It is preferable that the acoustic impedance of the filler layer 80 matches the acoustic impedance of the backing material layer 54 with given accuracy or higher such that the ultrasound signals propagated from the ultrasonic oscillator array 50 to the backing material layer 54 side are not reflected at a boundary surface between the filler layer 80 and the backing material layer 54. It is preferable that the filler layer 80 is made of a member having heat dissipation to increase efficiency in dissipating heat generated in a plurality of ultrasonic oscillators 48. In a case where the filler layer 80 has heat dissipation, since heat is received from the backing material layer 54, the FPC 60, the coaxial cables 56, and the like, heat dissipation efficiency can be improved.

With the ultrasonic oscillator unit 46 configured as described above, in a case where each ultrasonic oscillator 48 of the ultrasonic oscillator array 50 is driven, and a voltage is applied to the electrode 52 of the ultrasonic oscillator 48, the piezoelectric body 49 oscillates to sequentially generate ultrasonic waves, and the irradiation of the ultrasonic waves is performed toward the observation target part of the subject. Then, as a plurality of ultrasonic oscillators 48 are sequentially driven by an electronic switch, such as a multiplexer, scanning with ultrasonic waves is performed in a scanning range along a curved surface on which the ultrasonic oscillator array 50 is disposed, for example, a range of about several tens mm from the center of curvature of the curved surface.

In a case where the echo signal reflected from the observation target part is received, the piezoelectric body 49 vibrates to generate a voltage and outputs the voltage as an electric signal corresponding to the received ultrasound echo to the ultrasound processor device 14. Then, the electric signal is subjected to various kinds of signal processing in the ultrasound processor device 14 and is displayed as an ultrasound image on the monitor 20.

By the way, the FPC 60 shown in Fig. 4 has a plurality of electrode pads 62 that are electrically connected to a plurality of individual electrode 52a at one end, and a plurality of electrode pads 64 that are electrically connected to a plurality of signal lines 56a of the coaxial cable 56 at the other end. The FPC 60 also has a ground portion (not shown) that is electrically connected to the oscillator ground 52b.

Here, as shown in Fig. 3, the coaxial cables 56 of the present example are bundled using an outer coat 58 on the proximal end side of the distal end part 40, and are led out from the outer coat 58 and are connected to the FPC 60 at the time of wiring. As shown in a sectional view of Fig. 5, each coaxial cable 56 comprises a signal line 56a connected to the electrode pad 64 on a center side, and has an insulating outer coat 56b provided in a layer outside the signal line 56a, a shield layer 56c provided in a layer outside the outer coat 56b, and an insulating outer coat 56d provided in an outermost layer.

Returning to Fig. 4, an electrical bonded portion 100 of the electrode pad 62 and the individual electrode 52a is bonded by a conductive resin material 102, and an electrical bonded portion 104 of the electrode pad 64 and the signal line 56a is also boned by the conductive resin material 102.

Examples of the above-described resin material 102 include an anisotropic conductive film (ACF) or an anisotropic conductive paste (ACP) obtained by mixing thermosetting resin with fine conductive particles and forming the mixture into a film. The electrical bonded portions 100 and 104 bonded by such a resin material 102 may be deteriorated and disconnected due to contact with sterilizing gas, such as ethylene oxide gas or hydrogen peroxide plasma gas, in a case where the ultrasonic endoscope 12 is cleaned by a gas sterilization device.

Accordingly, in the ultrasonic endoscope 12 of the embodiment, as shown in Fig. 4, each of the electrical bonded portions 100 and 104 is coated with a low reactive epoxy resin layer 106 that is a first resin layer. Then, even though the above-described sterilizing gas is transmitted through the acoustic lens 78 or the filler layer 80 and enters the electrical bonded portions 100 and 104 side, since the above-described epoxy resin layer 106 exhibits a function as a gas barrier, it is possible to suppress contact of the sterilizing gas with the electrical bonded portions 100 and 104. With this, it is possible to suppress deterioration of the electrical bonded portions 100 and 104 due to the sterilizing gas.

Accordingly, with the ultrasonic endoscope 12 of the embodiment, since the electrical bonded portions 100 and 104 from the coaxial cable 56 to the piezoelectric body 49 are bonded by the conductive resin material 102, and the electrical bonded portions 100 and 104 using the resin material 102 are coated with the gas barrier epoxy resin layer 106, it is possible to suppress deterioration of the electrical bonded portions 100 and 104 due to the sterilizing gas.

Although it is preferable that all electrical bonded portions from the coaxial cable 56 to the piezoelectric body 49 are coated with the epoxy resin layer 106, the present invention is not limited thereto, at least the electrical bonded portions 100 and 104 that are bonded by the conductive resin material 102 may be coated with the epoxy resin layer 106.

In the ultrasonic endoscope 12 of the embodiment, although an aspect where the present example is applied to the connection structure in which the piezoelectric bodies 49 and the coaxial cables 56 are connected through the FPC 60 has been described, a connection structure to which the present example is applied is not limited thereto. For example, the present example can be applied to a first another connection structure in which the piezoelectric bodies 49 and the coaxial cables 56 are connected through electrical bonded portions, and a second another connection structure in which a first FPC connected to the piezoelectric bodies 49 and a second FPC connected to the coaxial cables 56 are connected through electrical bonded portions. In this case, in the second another connection structure, since stress is likely to be concentrated on the above-described electrical bonded portions at the time of handling during manufacturing, it is preferable that the electrical bonded portions are coated with the epoxy resin layer 106 in advance. With this, since mechanical strength of the electrical bonded portions is improved, it is possible to restrain the electrical bonded portions from being damaged at the time of handling during manufacturing.

In the ultrasonic endoscope 12 of the embodiment, although the resin material 102 is the ACF, the present invention is not limited thereto. For example, a resin material in which a conductive filler, such as metallic particles, is dispersed into binder resin, such as epoxy or urethane, and the conductive filler forms a conductive path after adhesion may be used. Examples of this resin material include a conductive paste, such as a silver paste.

Hereinafter, a specific example of the epoxy resin layer 106 will be described.

It is preferable that the epoxy resin of the epoxy resin layer 106 has a polyoxyalkylene structure. With this, gas resistance of the epoxy resin layer 106 to the sterilizing gas is improved.

It is preferable that the epoxy resin of the epoxy resin layer 106 contains an alcohol compound. With this, gas resistance of the epoxy resin layer 106 to the sterilizing gas is improved. In this case, it is more preferable that a hydroxyl group equivalent of the alcohol compound is equal to or greater than 25 and equal to or less than 150, and a molecular weight of the alcohol compound is equal to or greater than 50 and equal to or less than 500. With this, it is possible to obtain the same gas resistance as the above-described polyoxyalkylene structure.

It is preferable that the epoxy resin of the epoxy resin layer 106 has a polyamide structure. With this, gas resistance of the epoxy resin layer 106 to the sterilizing gas is improved.

On the other hand, it is preferable that second resin for forming the filler layer 80 is epoxy resin, and it is more preferable that the epoxy resin has a polyamide structure. With this, it is possible to allow the filler layer 80 to have gas resistance.

Here, epoxy resin having high gas resistance (that is, low responsiveness to gas) has a large molecular weight and high viscosity. From such situations, it is preferable that epoxy resin having high viscosity before curing to some extent is employed as the first resin layer with which the electrical bonded portions 100 and 104 are coated. With this, it is possible to increase gas resistance, and to reliably coat the electrical bonded portions 100 and 104.

It is preferable that epoxy resin having lower viscosity before curing than the first resin is employed as the second resin for forming the filler layer 80. With this, since it is possible to spread the second resin over the entire region of the internal space 55, it is possible to suppress the occurrence of air bubbles that cause gas transmission, in the filler layer 80.

It is preferable that the viscosity before curing of the first resin layer is equal to or greater than 50 Pa·s and equal to or less than 500 Pa·s as an example. The viscosity before curing of the second resin layer is preferably equal to or greater than 1 Pa·s and equal to or less than 30 Pa·s, and is more preferably equal to or greater than 1 Pa·s and equal to or less than 15 Pa·s.

By the way, since the ultrasonic endoscope is inserted into a human body, there is a need for a reduction in diameter of the insertion part. To realize the reduction in diameter of the insertion part, the cable that is connected to the ultrasonic oscillator is very fine compared to a cable that is used in a body surface echo. As a result, there is a problem in that mechanical strength of the electrical bonded portions is very weak, and the electrical bonded portions are easily damaged at the time of handling during manufacturing. In particular, since a load is applied to the cable at the time of work of storing the ultrasonic oscillator in the distal end part, there is a high possibility that the electrical bonded portions are damaged.

In a case where solder, instead of a conductive resin material, is used in bonding for a small ultrasonic oscillator that is employed in the ultrasonic endoscope, and in a case where heat equal to or higher than 100 degrees is transmitted to the piezoelectric body, the piezoelectric body has an increased risk of being damaged due to the occurrence of microcracks in the piezoelectric body. Alternatively, a disposition interval of the electrode pads of the FPC is narrow and bonding work is impossible with solder, and a solder defect is likely to occur. For this reason, a place where bonding needs to be performed using a conductive resin material necessarily occurs. In this case, since the conductive resin material has weak mechanical strength compared to solder, there is a higher possibility that the electrical bonded portions are damaged at the time of handling during manufacturing. In this way, an ultrasonic endoscope of the related art has a problem that the electrical bonded portions are easily damaged at the time of handling during manufacturing.

In contrast, in the ultrasonic endoscope 12 of the embodiment, since the electrical bonded portions 100 and 104 are coated with the epoxy resin layer 106, mechanical strength of the electrical bonded portions 100 and 104 is improved. With this, the ultrasonic endoscope 12 of the embodiment solves the problem that the electrical bonded portions 100 and 104 are easily damaged at the time of handling during manufacturing.

Although the present invention has been described, the present invention is not limited to the above-described example, and various improvements or modifications may be of course made without departing from the scope of the present invention.

### Explanation of References

10: ultrasonography system
12: ultrasonic endoscope
14: ultrasound processor device
16: endoscope processor device
18: light source device
20: monitor
21a: water supply tank
21b: suction pump
22: insertion part
24: operating part
26: universal cord
29: angle knob
30: treatment tool insertion port
32a: connector
32b: connector
32c: connector
34a: air and water supply tube
34b: suction tube
36: ultrasonic observation part
38: endoscope observation part
40: distal end part
41: exterior member
42: bendable part
43: soft part
44: treatment tool outlet port
45: treatment tool channel
46: ultrasonic oscillator unit
48: ultrasonic oscillator
49: piezoelectric body
50: ultrasonic oscillator array
52: electrode
52a: individual electrode
52b: oscillator ground
54: backing material layer
55: internal space
56: coaxial cable
56a: signal line
56b: outer coat
56c: shield layer
56d: outer coat
58: outer coat
60: FPC
62: electrode pad
64: electrode pad
76: acoustic matching layer
78: acoustic lens
80: filler layer
82: observation window
84: objective lens
86: solid-state imaging element
88: illumination window
90: cleaning nozzle
92: wiring cable
100: electrical bonded portion
102: resin material
104: electrical bonded portion
106: epoxy resin layer

## Claims

1. An ultrasonic oscillator unit (46) that is disposed in a distal end part (40) of an endoscope insertion part (22) and has a plurality of ultrasonic oscillators (48),
wherein the ultrasonic oscillators each have a piezoelectric body (49),
a cable (92) that is electrically bonded to the piezoelectric body (49) is inserted into an internal space (55) of the distal end part (40),
at least one of a plurality of electrical bonded portions (100,104) from the cable (92) to the piezoelectric body (49) is bonded by a resin material (102) having conductivity,
the at least one of the plurality of electrical bonded portions using the resin material (102) is covered with a first resin layer (106) whose material is different from that of the resin material (102), and
the first resin layer is made of gas barrier epoxy resin (106).

2. The ultrasonic oscillator unit (46) according to claim 1,
wherein the epoxy resin has a polyoxyalkylene structure.

3. The ultrasonic oscillator unit (46) according to claim 1 or 2,
wherein the epoxy resin contains an alcohol compound.

4. The ultrasonic oscillator unit (46) according to claim 3,
wherein a hydroxyl group equivalent of the alcohol compound is equal to or greater than 25 and equal to or less than 150, and a molecular weight of the alcohol compound is equal to or greater than 50 and equal to or less than 500.

5. The ultrasonic oscillator unit (46) according to any one of claims 1 to 3,
wherein the epoxy resin has a polyamide structure.

6. The ultrasonic oscillator unit (46) according to any one of claims 1 to 5,
wherein the internal space (55) of the distal end part (40) into which the cable (92) is inserted is filled with a second resin layer, and
the second resin layer is made of epoxy resin.

7. The ultrasonic oscillator unit (46) according to claim 6,
wherein the epoxy resin has a polyamide structure.

8. The ultrasonic oscillator unit (46) according to claim 6 or 7,
wherein the first resin layer (106) has higher viscosity before curing than the second resin layer.

9. An ultrasonic endoscope (12) comprising:
an insertion part (22) that is configured to be inserted into a body;
an ultrasonic observation part (36) provided at a distal end of the insertion part (22); and
the ultrasonic oscillator unit (46) according to any one of claims 1 to 8 provided in the ultrasonic observation part (36).

## Patentansprüche

1. Ultraschalloszillatoreinheit (46), die in einem distalen Endteil (40) eines Endoskopeinführteils (22) angeordnet ist und mehrere Ultraschalloszillatoren (48) aufweist,
wobei die Ultraschalloszillatoren jeweils einen piezoelektrischen Körper (49) aufweisen,
ein Kabel (92), das mit dem piezoelektrischen Körper (49) elektrisch gebunden ist, in einen Innenraum (55) des distalen Endteils (40) eingeführt wird,
mindestens einer von mehreren elektrisch gebundenen Abschnitten (100, 104) von dem Kabel (92) zu dem piezoelektrischen Körper (49) durch ein Harzmaterial (102) mit Leitfähigkeit gebunden ist,
der mindestens eine von den mehreren elektrisch gebundenen Abschnitten unter Verwendung von dem Harzmaterial (102) mit einer ersten Harzschicht (106) abgedeckt ist, deren Material von dem des Harzmaterials (102) verschieden ist, und
die erste Harzschicht aus Gasbarriere-Epoxidharz (106) hergestellt ist.

2. Ultraschalloszillatoreinheit (46) nach Anspruch 1,
wobei das Epoxidharz eine Polyoxyalkylenstruktur aufweist.

3. Ultraschalloszillatoreinheit (46) nach Anspruch 1 oder 2,
wobei das Epoxidharz eine Alkoholverbindung enthält.

4. Ultraschalloszillatoreinheit (46) nach Anspruch 3,
wobei ein Hydroxylgruppenäquivalent der Alkoholverbindung gleich oder größer als 25 und gleich oder kleiner als 150 ist und ein Molekulargewicht der Alkoholverbindung gleich oder größer als 50 und gleich oder kleiner als 500 ist.

5. Ultraschalloszillatoreinheit (46) nach einem der Ansprüche 1 bis 3,
wobei das Epoxidharz eine Polyamidstruktur aufweist.

6. Ultraschalloszillatoreinheit (46) nach einem der Ansprüche 1 bis 5,
wobei der Innenraum (55) des distalen Endteils (40), in den das Kabel (92) eingeführt wird, mit einer zweiten Harzschicht gefüllt ist, und
die zweite Harzschicht aus Epoxidharz hergestellt ist.

7. Ultraschalloszillatoreinheit (46) nach Anspruch 6,
wobei das Epoxidharz eine Polyamidstruktur aufweist.

8. Ultraschalloszillatoreinheit (46) nach Anspruch 6 oder 7,
wobei die erste Harzschicht (106) vor Aushärtung höhere Viskosität als die zweite Harzschicht aufweist.

9. Ultraschallendoskop (12), umfassend:
einen Einführteil (22), der so konfiguriert ist, dass er in einen Körper eingeführt wird;
einen Ultraschallbeobachtungsteil (36), der an einem distalen Ende des Einführteils (22) vorgesehen ist; und
die Ultraschalloszillatoreinheit (46) nach einem der Ansprüche 1 bis 8, die bei dem Ultraschallbeobachtungsteil (36) vorgesehen ist.

## Revendications

1. Unité d'oscillation ultrasonore (46) qui est disposée dans une partie d'extrémité distale (40) d'une partie d'insertion d'endoscope (22) et a une pluralité d'oscillateurs ultrasonores (48),
dans lequel les oscillateurs ultrasonores ont chacun un corps piézoélectrique (49),
un câble (92) qui est électriquement relié au corps piézoélectrique (49) est inséré dans un espace interne (55) de la partie d'extrémité distale (40),
au moins une d'une pluralité de parties de liaison électrique (100,104) entre le câble (92) et le corps piézoélectrique (49) est reliée par un matériau de résine (102) ayant une conductivité,
au moins l'une de la pluralité de parties de liaison électrique utilisant le matériau de résine (102) est recouverte d'une première couche de résine (106) dont le matériau est différent de celui du matériau de résine (102), et
la première couche de résine est en résine époxy à barrière gazeuse (106).

2. Unité d'oscillation ultrasonore (46) selon la revendication 1,
dans lequel la résine époxy a une structure de polyoxyalkylène.

3. Unité d'oscillation ultrasonore (46) selon la revendication 1 ou la revendication 2, dans lequel la résine époxy contient un composé alcoolique.

4. Unité d'oscillation ultrasonore (46) selon la revendication 3,
dans lequel un équivalent de groupe hydroxyle du composé alcoolique est égal ou supérieur à 25 et égal ou inférieur à 150, et un poids moléculaire du composé alcoolique est égal ou supérieur à 50 et égal ou inférieur à 500.

5. Unité d'oscillation ultrasonore (46) selon l'une quelconque des revendications 1 à 3, dans lequel la résine époxy a une structure de polyamide.

6. Unité d'oscillation ultrasonore (46) selon l'une quelconque des revendications 1 à 5,
dans lequel l'espace interne (55) de la partie d'extrémité distale (40) dans lequel le câble (92) est inséré est rempli d'une deuxième couche de résine, et
la deuxième couche de résine est en résine époxy.

7. Unité d'oscillation ultrasonore (46) selon la revendication 6,
dans lequel la résine époxy a une structure de polyamide.

8. Unité d'oscillation ultrasonore (46) selon la revendication 6 ou la revendication 7, dans laquelle la première couche de résine (106) a une viscosité plus élevée avant durcissement que la deuxième couche de résine.

9. Endoscope ultrasonore (12) comprenant :
une partie d'insertion (22) qui est configurée pour être insérée dans un corps ;
une partie d'observation ultrasonore (36) prévue à une extrémité distale de la partie d'insertion (22) ; et
l'unité d'oscillation ultrasonore (46) selon l'une quelconque des revendications 1 à 8 prévue dans la partie d'observation ultrasonore (36).
